(19) Europäisches Patentamt
European Patent Office
Office européen des brevets

(11) Veröffentlichungsnummer : **0 023 598**
**B1**

# (12) EUROPÄISCHE PATENTSCHRIFT

(45) Veröffentlichungstag der Patentschrift : **24.02.82**

(21) Anmeldenummer : **80103924.9**

(22) Anmeldetag : **09.07.80**

(51) Int. Cl.³ : **C 07 C 21/073**, C 07 C 17/28, C 07 C 21/24, C 07 C 25/28, C 07 C 45/68, C 07 C 49/567, C 07 C120/00, C 07 C121/48, C 07 C 1/34// C07F9/40

## (54) Verfahren zur Herstellung von 1,1-Dichloralkenen.

(30) Priorität : **21.07.79 DE 2929645**

(43) Veröffentlichungstag der Anmeldung : **11.02.81 (Patentblatt 81/06)**

(45) Bekanntmachung des Hinweises auf die Patenterteilung : **24.02.82 Patentblatt 82/08**

(84) Benannte Vertragsstaaten : **BE CH DE FR GB IT LI NL**

(56) Entgegenhaltungen :
**EP - A1 - 0 000 345**
**EP - A1 - 0 002 849**
**EP - A1 - 0 011 695**
**DE - A1 - 2 827 101**
**Chemical Abstracts Band 83, Nr. 23, 8. Dezember 1975 Columbus, Ohio, USA**
**P. SAVIGNAC et al. « The α-chlorination and carbonyl olefination » Seite 399, Spalte 2, Abstract Nr. 192632r**

(73) Patentinhaber : **BAYER AG**
**Zentralbereich Patente, Marken und Lizenzen**
**D-5090 Leverkusen 1, Bayerwerk (DE)**

(72) Erfinder : **Hoffmann, Hellmut, Dr. Prof.**
**Tersteegenweg 17**
**D-5600 Wuppertal (DE)**
Erfinder : **Maurer, Fritz, Dr.**
**Roeberstrasse 8**
**D-5600 Wuppertal (DE)**
Erfinder : **Priesnitz, Uwe, Dr.**
**Heinrich-Heine-Strasse 42**
**D-4750 Unna-Massen (DE)**
Erfinder : **Riebel, Hans-Jochem, Dr.**
**In der Beek 92**
**D-5600 Wuppertal (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 023 598 B1

Imprimerie Jouve, 18, rue St-Denis, 75001 Paris, France

# Verfahren zur Herstellung von 1,1-Dichloralkenen

Die Erfindung betrifft ein neues Verfahren zur Herstellung von 1,1-Dichlor-alkenen.

Es ist bekannt, daß man 1,1-Dichloralkene, wie z.B. 3-(2,2-Dichlor-vinyl)-2,2-dimethyl-cyclopropan-1-carbonsäure-tert.-butylester, erhält, wenn man Aldehyde, wie z.B. 3-Formyl-2,2-dimethyl-cyclopropan-1-carbonsäure-tert.-butylester mit Triphenylphosphin in Tetrachlorkohlenstoff umsetzt (vergleiche DE-OS 2 326 077). Nach dieser Synthesemethode erhält man die gewünschten Produkte jedoch nur in mäßigen Ausbeuten.

Es ist weiter bekannt, daß man 1,1-Dichlor-alkene erhält, wenn man Lithiumsalze von Dichlormethanphosphonsäureestern mit Aldehyden oder Ketonen umsetzt (vergleiche Synthesis *1975*, 535-536).

Die Herstellung der Lithiumsalze von Dichlormethanphosphonsäureestern ist jedoch relativ aufwendig ; man erhält sie aus Chlormethan-phosphonsäureestern durch Umsetzung mit Beryllithium und Tetrachlorkohlenstoff bei − 75 °C, wobei sorgfältig getrocknete Lösungsmittel zu verwenden sind und eine Inertgasatmosphäre erforderlich ist.

Es wurde nun ein Verfahren zur Herstellung von 1,1-Dichloralkenen der Formel I

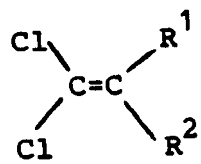

(I)

gefunden, in welcher

$R^1$ für Wasserstoff, oder für einen gegebenenfalls substituierten Alkyl-, Alkenyl-, Alkinyl-, Aralkyl-, oder Aryl-Rest steht und

$R^2$ für einen gegebenenfalls substituierten Alkyl-, Alkenyl-, Alkinyl-, Aralkyl-, Aralkenyl- oder Aryl-Rest oder für gegebenenfalls durch Halogen, Alkyl, Alkanoyl, Carbamoyl, Cyano oder Phenyl substituiertes Cycloalkyl steht, oder in welcher die beiden Reste $R^1$ und $R^2$ zusammen für eine gegebenenfalls verzweigte und/oder gegebenenfalls benzannellierte Kohlenwasserstoffkette stehen,

dadurch gekennzeichnet, daß man Aldehyde oder Ketone der Formel II

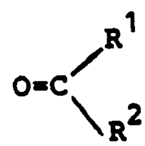

(II)

in welcher

$R^1$ und $R^2$ die oben angegebenen Bedeutungen haben,

mit Dichlormethan-phosphonsäureestern der Formel III

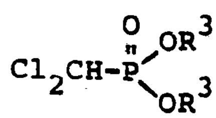

(III)

in welcher

$R^3$ einzeln für Alkyl oder Phenyl steht oder beide Reste $R^3$ zusammen für Alkandiyl (Alkylen) stehen, in Gegenwart einer Base und gegebenenfalls unter Verwendung eines Verdünnungsmittels bei Temperaturen zwischen − 50 und + 50 °C umsetzt.

Es ist als überraschend anzusehen, daß man nach dem erfindungsgemäßen Verfahren 1,1-Dichloralkene der Formel (I) wesentlich einfacher und kostengünstiger in guten Ausbeuten erhält als man in Anbetracht des Standes der Technik erwarten konnte.

Es war ferner überraschend, daß es bei dieser Reaktion auf die Reihenfolge anzukommen scheint, in der die Reaktionspartner zusammengegeben werden.

Während man diese Art der Reaktion üblicherweise so durchführen würde, daß man zum Phosphonsäureester der Formel III eine Base zufügt und anschließend die Reaktionslösung mit den Aldehyden oder Ketonen der Formel II versetzt, zeigt sich, daß das erfindungsgemäße Verfahren gerade bei dieser Arbeitsweise meist nicht oder nur schlecht durchführbar ist. Aus diesem Grund sind Verfahrensweisen bevorzugt bei denen entweder die Base zum Gemisch der Verbindungen II und III zugetropft wird, oder die Verbindung III zum Gemisch aus Base und Verbindung II zugegeben wird.

Verwendet man als Ausgangsstoffe beispielsweise 3-Formyl-2,2-Dimethyl-cyclopropan-1-carbonsäure-nitril und Dichlormethan-phosphonsäure-dimethyl-ester sowie als Base Natriummethylat, so kann die Reaktion dieser Verbindungen durch folgendes Formelschema skizziert werden :

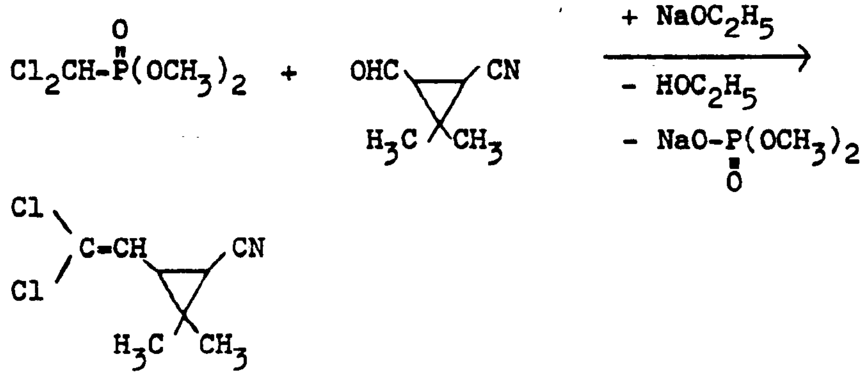


Die als Ausgangsstoffe zu verwendenden Aldehyde bzw. Ketone sind durch Formel (II) definiert. Vorzugsweise stehen darin

$R^1$ für Wasserstoff, für gegebenenfalls halogen-substituiertes $C_1$-$C_5$-Alkyl, für gegebenenfalls halogen-substituiertes Benzyl oder Phenyläthyl, für gegebenenfalls durch Halogen, $C_1$-$C_4$-Alkyl, $C_1$-$C_4$-Alkoxy, Methylendioxy, Cyano und/oder Nitro substituiertes Phenyl,

$R^2$ gegebenenfalls halogen-substituiertes $C_1$-$C_5$-Alkyl, für $C_2$-$C_5$-Alkenyl, für $C_2$-$C_5$-Alkinyl, für gegebenenfalls halogen-substituiertes Benzyl oder Phenyläthyl, für gegebenenfalls durch Halogen, $C_1$-$C_4$-Alkyl, $C_1$-$C_4$-Alkoxy, Methylendioxy, Cyano und/oder Nitro substituiertes Phenyl, für gegebenenfalls halogen-substituiertes Styryl oder für den Rest

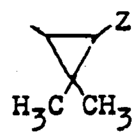

wobei Z für Acetyl, Cyano oder Carbamoyl steht, oder beide Reste stehen zusammen für geradkettiges oder verzweigtes Alkandiyl mit 4 bis 10 Kohlenstoffatomen.

Als Ausgangsstoffe besonders bevorzugt sind diejenigen Reste der Formel (II), in welcher

$R^1$ für Wasserstoff steht und

$R^2$ für $C_2$-$C_5$-Alkenyl oder den Rest

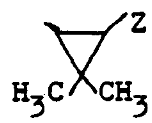

steht, worin Z für Cyano, Acetyl oder Carbamoyl steht.

Als Beispiele für die Ausgangsstoffe der Formel (II) seien genannt :

β,β-Dimethylacrolein, 3-Formyl-2,2-dimethyl-1-cyanocyclopropan,

3-Formyl-2,2-dimethyl-1-carbamoyl-cyclopropan und 3-Formyl-2,2-dimethyl-1-acetyl-cyclopropan.

Die Verbindungen der Formel (II) sind teilweise bekannt (vergleiche Synthesis *1975,* 535-536 ; Tetrahedron Lett. *1976,* 1979-1982). Die besonders bevorzugten Cyclopropan-derivate der Formel (II) sind teilweise noch nicht in der Literatur beschrieben. Man erhält diese Verbindungen nach an sich bekannten Verfahren. Ein Syntheseweg ist in nachstehendem Formelschema skizziert :

(R steht für $C_1$-$C_4$-Alkyl)

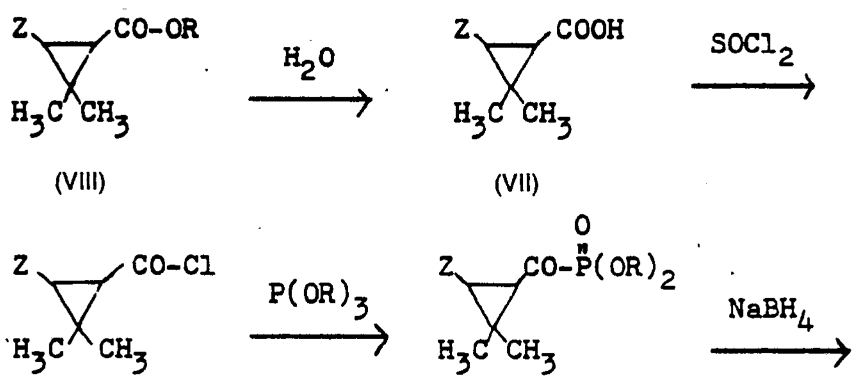


(VIII) (VII)

(VI) (V)

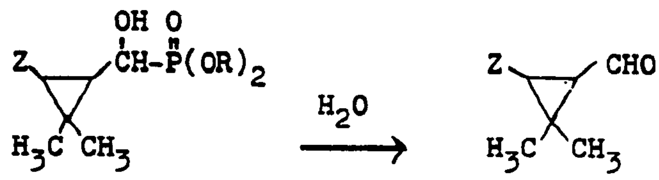

(IV) (II a)

Durch Hydrolyse von bekannten Cyclopropancarbonsäure-estern der Formel (VIII) (vergleiche J. Org. Chem. *32,* (1967), 3351-3355 ; Bull. soc. Chim. Belg. *87* (1978), 721-732 ; Tetrahedron Lett. *1978,* 1847-1850), beispielsweise durch Umsetzung mit wässrig-alkoholischer Kalilauge bei Temperaturen zwischen 20 und 100 °C und anschließendes Ansäuern erhält man die Carbonsäuren der Formel (VII), Diese können durch Umsetzung mit Halogenierungsmitteln, wie z.B. Thionylchlorid, bei Temperaturen zwischen 20 und 80 °C in die Säurechloride der Formel (VI) umgewandelt werden.

Durch Umsetzung der Säurechloride (VI) mit Trialkylphosphiten bei Temperaturen zwischen −20 und +150 °C, vorzugsweise zwischen 0 und 120 °C erhält man die Cyclopropanoylphosphonsäureester der Formel (V) (vergleiche J. Am. Chem. Soc. *86* (1964), 3862-3866 ; Methoden der organischen Chemie (Houben-Weyl-Müller), 4. Auflage, Band *12/1,* S. 453, Georg-Thieme-Verlag, Stuttgart 1963). Zur Isolierung und Reinigung der Produkte wird, gegebenenfalls unter vermindertem Druck destilliert.

Die α-Hydroxy-phosphonsäureester der Formel (IV) erhält man durch Reduktion der Oxoverbindungen der Formel (V) mit Natriumtetrahydridoborat, gegebenenfalls unter Verwendung eines Verdünnungsmittels, wie z.B. Wasser oder wässr. Methanol, bei Temperaturen zwischen −20 und +50 °C und Halten des PH-Wertes zwischen 5 und 8 durch Zugabe eines Puffermittels, wie z.B. Natriumhydrogenphosphat (vergleiche Chem. Ber. *103,* (1970), 2984-2986). Zur Aufarbeitung extrahiert man mit einem mit Wasser nicht mischbaren Lösungsmittel, wie z.B. Methylenchlorid, trocknet die Extrakte, filtriert und destilliert das Lösungsmittel unter vermindertem Druck ab.

Aus den α-Hydroxy-phosphonsäureestern der Formel (IV) können die entsprechenden Aldehyde der Formel (II a) durch Behandeln mit Natronlauge bei Temperaturen zwischen 0 und 100 °C, vorzugsweise zwischen 10 und 50 °C hergestellt werden (vergleiche Chem. Ber. *103* (1970), 2 984-2 986).

Alternativ zum oben skizzierten Herstellungsverfahren erhält man Aldehyde der Formel (II a) auch durch Umsetzung von Säurechloriden der Formel (VI) mit Lithium-tri-tert.-butoxy-hydrido-aluminat, welches man gegebenenfalls in situ aus Lithium-tetrahydridoaluminat und tert.-Butanol hergestellt hat, gegebenenfalls in Gegenwart eines Verdünnungsmittels, wie z.B. Tetrahydrofuran, bei Temperaturen zwischen −100 und +100 °C, vorzugsweise zwischen −80 und +50 °C. Zur Aufarbeitung wird in eine Mischung aus Salzsäure und Eiswasser gegossen und mit einem mit Wasser nicht mischbaren Lösungsmittel, wie z.B. Diethylether, extrahiert. Die Extrakte werden getrocknet, filtriert und eingeengt. Zur Reinigung des Rohproduktes wird gegebenenfalls destilliert.

Die weiter als Ausgangsstoffe einzusetzenden Dichlormethan-phosphonsäureester sind durch Formel (III) definiert. Vorzugsweise steht darin

$R^3$ für Alkyl mit 1 bis 4 Kohlenstoffatomen, für Phenyl, oder beide Reste $R^3$ stehen zusammen für geradkettiges oder verzweigtes Alkandiyl (Alkylen) mit 2 bis 5 Kohlenstoffatomen.

Als Beispiele seien Dichlormethan-phosphonsäure-dimethylester, -diethylester und -diphenylester genannt.

Die Verbindungen der Formel (III) sind bekannt oder können nach an sich bekannten Verfahren hergestellt werden (vergleiche Synthesis *1975,* 535-536 ; Tetrahedron Lett. *1975,* 609-610 ; ibid. *1975,* 4 409-4 410).

Man erhält Dichlormethan-phosphonsäureester der Formel (III) beispielsweise durch Umsetzung von Dichlormethan-phosphonsäure-dichlorid (vergleiche GB-PS 707 961) mit Natrium- oder Kaliumsalzen von Hydroxyverbindungen, wie z.B. Methanol, Ethanol, n- und iso-Propanol, n-, iso-, sek.- und tert.-Butanol, gegebenenfalls in Gegenwart eines Verdünnungsmittels, wie z.B. Toluol, bei Temperaturen zwischen 0 und 50 °C. Zur Reinigung der Produkte wird, gegebenenfalls nach Filtration, destilliert.

Das erfindungsgemäße Verfahren wird vorzugsweise unter Verwendung von Verdünnungsmitteln durchgeführt. Als solche kommen praktisch alle inerten organischen Lösungsmittel, insbesondere aprotisch polare Solventien in Frage. Hierzu gehören Ether, wie z.B. Glycoldimethylether, Diglycoldimethylether, Tetrahydrofuran und Dioxan, Carbonsäureamide, wie z.B. Dimethylformamid, Dimethylacetamid und N-Methyl-pyrrolidon, Sulfoxide und Sulfone, wie z.B. Dimethylsulfoxid und Tetramethylensulfon, Phosphorsäureamide, wie z.B. Hexamethylphosphorsäuretriamid, sowie Nitrile, wie z.B. Acetonitril und Propionitril.

Beim erfindungsgemäßen Verfahren können die in der organisch-chemischen Synthese üblichen Basen verwendet werden. Hierzu gehören insbesondere Alkalihydroxide, wie z.B. Natrium- und Kaliumhydroxid, Alkalialkoholate, wie z.B. Natrium- und Kalium-methylat, Natrium- und Kalium-ethylat, Natrium- und Kalium-iso-propylat, Natrium- und Kalium-tert.-butylat, Alkalihydride, wie z.B. Natriumhydrid,

Alkaliamide, wie z.B. Natriumamid, Alkyllithiumverbindungen, wie z.B. Butyllithium sowie Amine, wie z.B. Diazobicyclooctan, Diazobicyclononan und Diazobicycloundecen. Amine und Alkoholate werden als Basen besonders bevorzugt.

Die Reaktionstemperatur wird zwischen −50 und +50 °C, vorzugsweise zwischen −30 und +30 °C gehalten. Das Verfahren wird im allgemeinen bei Normaldruck durchgeführt.

Je Mol 3-Formyl-2,2-dimethyl-cyclopropan-1-carbonsäure-ester der Formel (II) werden 1 bis 1,5 Mol, vorzugsweise 1 bis 1,2 Mol Dichlormethan-phosphonsäureester der Formel (III) und 1 bis 1,5 Mol, vorzugsweise 1 bis 1,2 Mol Base eingesetzt.

In einer bevorzugten Ausführungsform des erfindungsgemäßen Verfahrens werden die Ausgangsstoffe der Formeln (II) und (III) in einem der oben angegebenen Verdünnungsmittel bei Temperaturen zwischen −30 und 0 °C vorgelegt und mit der Lösung der Base in einem der oben angegebenen Verdünnungsmittel tropfenweise versetzt. Dann läßt man das Reaktionsgemisch auf Raumtemperatur kommen und rührt einige Stunden nach.

Die Aufarbeitung erfolgt auf übliche Weise : man verdünnt das Reaktionsgemisch mit Wasser und extrahiert mit einem mit Wasser nicht mischbaren Lösungsmittel, wie z.B. Methylenchlorid. Die Extrakte werden mit verdünnter Natronlauge und dann mit Wasser gewaschen, getrocknet und filtriert. Vom Filtrat wird das Lösungsmittel abgezogen und das als Rückstand verbleibende Produkt durch Vakuumdestillation gereinigt. Zur Charakterisierung dient das NMR-Spectrum und der Siedepunkt.

Die nach dem erfindungsgemäßen Verfahren herzustellenden 1,1-Dichlor-alkene können als Zwischen- produkte zur Herstellung von insektizid und akarizid wirksamen Pyrethroiden verwendet werden (vergleiche DE-OS' 2 621 833, 2 751 610 und 2 810 098).

## Herstellungsbeispiele

### Beispiel 1

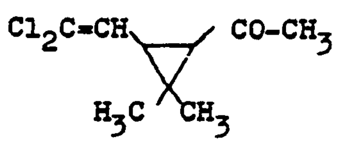

Zu einer Lösung von 24 g (0,108 Mol) Dichlormethanphosphonsäurediäthylester und 14 g (0,1 Mol) 2-Acetyl-3,3-dimethylcyclopropancarboxaldehyd in 100 ml Tetrahydrofuran tropft man unter Schutzgasatmosphäre (Stickstoff oder Argon) bei −20 bis −25 °C eine Lösung von 12,3 mg (0,11 Mol) Kalium-tert.-butylat in 30 ml Dimethylformamid. Das Gemisch wird 3 Stunden ohne Kühlung nachgerührt, mit 200 ml Wasser versetzt und 2 mal mit je 100 ml Methylenchlorid extrahiert. Die organische Phase wird mit 50 ml 10 %iger Natronlauge und dann 3 mal mit je 50 ml Wasser gewaschen, über Natriumsulfat getrocknet und im Vakuum eingedampft. Aus dem Rückstand erhält man durch Vakuumdestillation 14,8 g (71 % der Theorie) 1-(2,2-Dichlorvinyl)-2-acetyl-3,3-dimethylcyclopropan in Form eines schwach gelben Öles mit dem Siedepunkt 55-57 °C/0,3 mbar.

### Beispiel 2

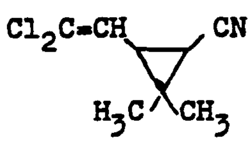

Analog Beispiel 1 kann aus 3-Formyl-2,2-dimethyl-cyclopropan-1-carbonsäurenitril und Dichlormethan-phosphonsäure-diethylester 3-(2,2-Dichlor-vinyl)-2,2-dimethylcyclopropan-1-carbonsäurenitril hergestellt werden. Ausbeute : 33 % der Theorie ; Siedepunkt 70 °C/0,1 mbar.

### Beispiel 3

$$(CH_3)_2C = CH—CH = C(Cl)_2$$

Zu einer Lösung von 16,8 g (0,2 Mol) Dimethylacrolein und 48,6 g (0,22 Mol) Dichlormethanphosphonsäurediethylester in 200 mol Tetrahydrofuran werden bei −15 °C 24,6 g (0,22 Mol) Kalium-tert.-butylat in 100 ml Tetrahydrofuran getropft. Man läßt die Reaktionstemperatur nach beendeter Zugabe auf 20 °C steigen und rührt 1 Stunde bei dieser Temperatur nach. Dann werden 500 ml Wasser zum Reaktionsgemisch gegeben, und es wird 2 mal mit je 150 ml Methylenchlorid extrahiert. Die vereinigten organischen Extrakte werden mit Wasser gewaschen, über Natriumsulfat getrocknet und fraktioniert.

Man erhält 26,4 g (87,5 % der Theorie) 1,1-Dichlor-4,4-dimethylbutadien in Form eines farblosen Öles mit dem Siedepunkt 52-54 °C/11 mbar.

Beispiel 4

$$(CH_3)_2C = CH—CH = C(Cl)_2$$

Es wird wie in Beispiel 3 gearbeitet. Kalium-tert.-butylat wird aber in 20 ml Dimethylformamid zugetropft. Man erhält 1,1-Dichlor-4,4-dimethyl-butadien in einer Ausbeute von 77,5 % der Theorie.

Beispiel 5

$$(CH_3)_2C = CH—CH = C(Cl)_2$$

Es wird wie in Beispiel 3 gearbeitet. Anstelle von (0,22 Mol) Kalium-tert.-butylat in 100 ml Tetrahydrofuran, das bei −15 °C zugetropft wird, werden aber 0,22 Mol Natriumethylat in 100 ml Tetrahydrofuran bei einer Zutropftemperatur von +15 °C verwendet. Man erhält 1,1-Dichlor-4,4-dimethylbutadien in einer Ausbeute von 48,8 % der Theorie.

Analog einem der Beispiele 1 bis 5 erhält man auch

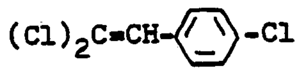

Siedepunkt 80 °C/0,3 mbar

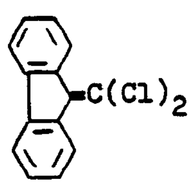

Schmelzpunkt 134 °C

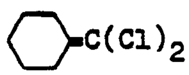

Siedepunkt 40 °C/0,5 mbar

Als Ausgangsstoffe zu verwendende Formyl-cyclopropan-derivate der Formel (II a) können beispielsweise wie folgt hergestellt werden :

Variante (a)

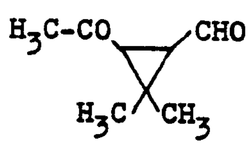

Zu einer Mischung aus 6,3 g (0,165 Mol) Lithiumalanat und 100 ml Tetrahydrofuran tropft man bei 20-30 °C innerhalb 1 Stunde 36,6 g (0,495 Mol) tert.-Butanol. Man rührt das Gemisch 2 Stunden bei Raumtemperatur nach und tropft es dann bei −50 bis −60 °C zu einer Lösung von 26,1 g (0,15 Mol) trans-3,3-Dimethyl-2-acetyl-cyclopropancarbonsäurechlorid in 75 ml Tetrahydrofuran. Nach Ende der Zugabe wird 1 Stunde ohne Kühlung nachgerührt, auf ein Gemisch aus 30 ml konzentriertem Natriumchlorid und 300 g Eis gegossen und 2 mal mit je 400 ml Ether ausgeschüttelt. Die organischen Phasen werden erst mit 50 ml gesättigter Natriumhydrogencarbonatlösung und dann mit 100 ml Wasser gewaschen, über Natriumsulfat getrocknet und im Vakuum eingedampft. Durch Destillation des Rückstandes im Vakuum erhält man 12,7 g (62 % der Theorie) trans-3,3-Dimethyl-2-acetyl-cyclopropancarboxaldehyd in Form eines farblosen Öles mit dem Siedepunkt 80-86 °C/10 mbar.

Variante (b)

Eine Mischung aus 50 g α-Hydroxy-α-(3-acetyl-2,2-dimethyl-cycloprop-1-yl)-methan-phosphonsäuredimethylester, 8 g Natriumhydroxid, 60 ml Wasser und 200 ml Methylenchlorid wird 90 Minuten bei Raumtemperatur gerührt. Die organische Phase wird abgetrennt, getrocknet und 2 mal destilliert. Man erhält 20 g (71 % der Theorie) 3,3-Dimethyl-2-acetyl-cyclopropancarboxaldehyd in Form eines farblosen Öles vom Siedepunkt 78 °C/8 mbar.

Als Ausgangsstoffe zu verwendende α-Hydroxy-phosphonsäureester der Formel (IV) können beispielsweise wie folgt hergestellt werden :

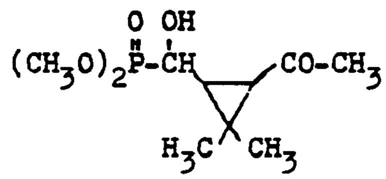

Eine Lösung von 50 g (0,2 Mol) α-Oxo-α-(3-acetyl-2,2-dimethyl-cycloprop-1-yl)-methanphosphonsäuredimethylester in 50 ml Methylenchlorid wird zu einer auf 0 bis 5 °C gekühlten Mischung aus 2,5 g Natriumtetrahydridoborat, 100 ml Wasser und 100 ml Methylenchlorid tropfenweise gegeben und das Reaktionsgemisch wird zwei Stunden bei 0 bis 5 °C gerührt. Dann wird die wässrige Phase von der organischen Phase abgetrennt und noch zweimal mit Methylenchlorid extrahiert. Die vereinigten organischen Phasen werden getrocknet, filtriert und eingedampft. Nach Umkristallisieren des zurückbleibenden Rohproduktes aus 200 ml Essigester/Ligroin (2 : 8) erhält man 31 g (62 % der Theorie) α-Hydroxy-α-(3-acetyl-2,2-dimethyl-cycloprop-1-yl)-methanphosphonsäuredimethylester vom Schmelzpunkt 104 °C.

Analog vorstehendem Beispiel erhält man

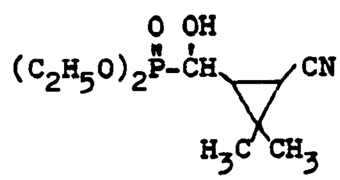

Als Ausgangsstoffe zu verwendende α-Oxo-phosphonsäure-ester der Formel (V) können beispielsweise wie folgt hergestellt werden :

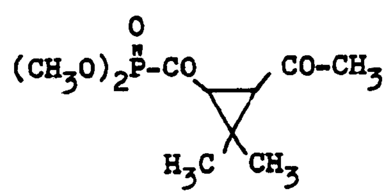

6,5 g (0,05 Mol) Trimethylphosphit werden zu einer auf 35 bis 40 °C erwärmten Lösung von 9 g (0,05 Mol) 3-Acetyl-2,2-dimethyl-cyclopropan-1-carbonsäurechlorid in 20 ml Methylenchlorid tropfenweise gegeben und das Reaktionsgemisch wird 15 Stunden bei 15 bis 25 °C gerührt. Nach Abdestillieren des Lösungsmittels im Vakuum erhält man 9 g (72 % der Theorie) α-Oxo-α-(3-acetyl-2,2-dimethylcycloprop-1-yl)-methan-phosphonsäure-dimethylester.

Analog vorstehendem Beispiel erhält man

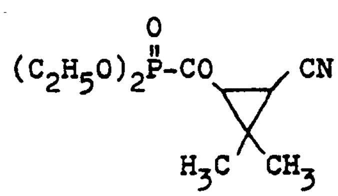

Als Ausgangsstoffe zu verwendende Cyclopropancarbonsäurechloride der Formel (VI) können beispielsweise wie folgt hergestellt werden :

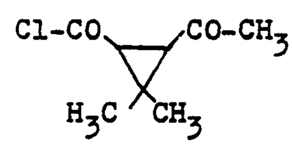

Eine Mischung aus 172 g 3-Acetyl-2,2-dimethyl-cyclopropan-1-carbonsäure, 130 g Thionylchlorid, 2 ml Dimethylformamid, und 200 ml Methylenchlorid wird vier Stunden unter Rückfluß zum Sieden erhitzt. Man erhält nach Destillation im Vakuum 135 g (71 % der Theorie) 3-Acetyl-2,2-dimethyl-cyclopropan-1-carbonsäurechlorid vom Siedepunkt 86 °C/15 mbar.

Analog erhält man

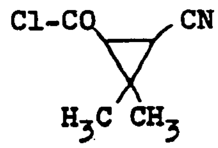

Als Ausgangsstoffe zu verwendende Dichlormethan-phosphonsäureester der Formel (III) können beispielsweise wie folgt hergestellt werden :

(a)

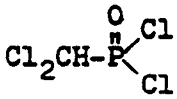

In eine Mischung aus 720 g (6 Mol) Chloroform und 274 g (2 Mol) Phosphortrichlorid werden portionsweise 266 g (2 Mol) Aluminiumchlorid gegeben. Danach wird das Reaktionsgemisch 12 Stunden unter Rückfluß zum Sieden erhitzt. Anschließend wird das Lösungsmittel im Vakuum abgezogen und der Rückstand wird in 2 l Methylenchlorid aufgenommen. Dazu werden bei 0 °C 440 ml konz. Salzsäure getropft und es wird etwa 2 Stunden bei dieser Temperatur nachgerührt. Die organische Phase wird abgetrennt, über Natriumsulfat getrocknet und eingeengt. Der Rückstand wird fraktioniert destilliert.

Man erhält 258 g (64,5 % der Theorie) Dichlormethanphosphonsäuredichlorid in Form eines farblosen Öls vom Siedepunkt 48-50 °C/1 mbar.

(b)

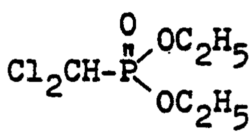

Zu einer Lösung von 227,5 g (1,13 Mol) Dichlormethanphosphonsäuredichlorid in 800 ml Toluol werden bei 5-10 °C 153,5 g (2,26 Mol) Natriumethylat in 800 ml Ethanol getropft. Es wird 5 Stunden bei 20 °C nachgerührt. Dann wird abgesaugt, das Filtrat eingeengt und der Rückstand fraktioniert destilliert. Man erhält 195 g (78 % der Theorie) Dichlormethanphosphonsäure-diethylester in Form eines farblosen Öls vom Siedepunkt 84 °C/1 mbar.

## Ansprüche

1. Verfahren zur Herstellung von 1,1-Dichloralkenen der Formel I

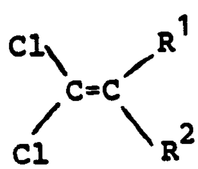 (I)

in welcher

$R^1$ für Wasserstoff, oder für einen gegebenenfalls substituierten Alkyl-, Alkenyl-, Alkinyl-, Aralkyl-, oder Aryl-Rest steht und

$R^2$ für einen gegebenenfalls substituierten Alkyl-, Alkenyl-, Alkinyl-, Aralkyl-, Arakenyl- oder Aryl-Rest oder für gegebenenfalls durch Halogen, Alkyl, Alkanoyl, Carbamoyl, Cyano, oder Phenyl substituiertes Cycloalkyl steht, oder in welcher die beiden Reste $R^1$ und $R^2$ zusammen für eine gegebenenfalls verzweigte und/oder gegebenenfalls benzannellierte Kohlenwasserstoffkette stehen,

dadurch gekennzeichnet, daß man Aldehyde oder Ketone der Formel II

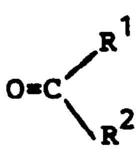 (II)

in welcher

$R^1$ und $R^2$ die oben angegebenen Bedeutungen haben, mit Dichlormethan-phosphonsäureestern der Formel III

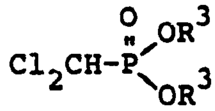

(III)

in welcher

$R^3$ einzeln für Alkyl oder Phenyl steht oder beide Rest $R^3$ zusammen für Alkandiyl (Alkylen) stehen, in Gegenwart einer Base und gegebenenfalls unter Verwendung eines Verdünnungsmittels bei Temperaturen zwischen −50 und +50 °C umsetzt.

2. Verfahren gemäß Anspruch 1, dadurch gekennzeichnet, daß man zum Gemisch der Verbindungen II und III die Base zudosiert.

**Claims**

1. Process for the preparation of 1,1-dichloralkenes of the formula I

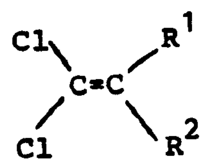

(I)

in which

$R^1$ represents hydrogen or an optionally substituted alkyl, alkenyl, alkynyl, aralkyl or aryl radical and

$R^2$ represents an optionally substituted alkyl, alkenyl, alkynyl, aralkyl, aralkenyl or aryl radical or cycloalkyl which is optionally substituted by halogen, alkyl, alkanoyl, carbamoyl, cyano or phenyl, or in which the two radicals $R^1$ and $R^2$ together represent a hydrocarbon chain which is optionally branched and/or optionally contains a fused benzene ring,

characterised in that aldehydes or ketones of the formula II

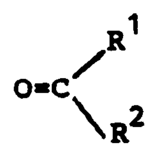

(II)

in which

$R^1$ and $R^2$ have the meanings given above, are reacted with dichloromethane-phosphonic acid esters of the formula III

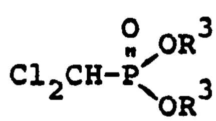

(III)

in which

$R^3$ individually represents alkyl or phenyl or the two radicals $R^3$ together represent alkanediyl (alkylene),

in the presence of a base and if appropriate using a diluent, at temperatures between −50 ° and +50 °C.

2. Process according to Claim 1, characterised in that the base is metered into a mixture of the compounds II and III.

**Revendications**

1. Procédé de préparation de 1,1-dichloralcènes de formule I

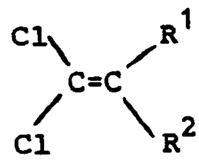 (I)

dans laquelle

$R^1$ représente l'hydrogène ou un groupe alkyle, alcényle, alcynyle, aralkyle ou aryle éventuellement substitué et

$R^2$ représente un reste alkyle, alcényle, alcynyle, aralkyle, aralcényle ou aryle éventuellement substitué ou un groupe cycloalkyle éventuellement substitué par des halogènes, des groupes alkyle, alcanoyle, carbamoyle, cyano ou phényle, ou bien encore les deux symboles $R^1$ et $R^2$ représentent ensemble une chaîne hydrocarbonée éventuellement ramifiée et/ou éventuellement cyclique benzénique condensée,

caractérisé en ce que l'on fait réagir des aldéhydes ou cétones de formule II

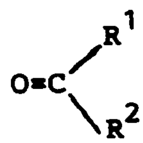 (II)

dans laquelle

$R^1$ et $R^2$ ont les significations indiquées ci-dessus, avec des esters d'acides dichlorométhane-phosphoniques de formule III

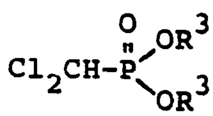 (III)

dans laquelle

chacun des symboles $R^3$ représente un groupe alkyle ou phényle ou bien les deux symboles $R^3$ représentent ensemble un groupe alcane-diyle (alkylène),

en présence d'une base et, le cas échéant, avec utilisation d'un diluant, à des températures comprises entre −50 et +50 °C.

2. Procédé selon la revendication 1, caractérisé en ce que l'on ajoute la base au mélange des composés II et III.